# EUROPEAN PATENT APPLICATION

(11) **EP 4 635 482 A1**
(43) Date of publication of application: **22.10.2025**
(21) Application number: 25169624.1
(22) Date of filing: 10.04.2025
(51) Int. Cl.: A61K 9/14, A61K 47/38, A61K 31/4422, A61K 9/20, A61K 9/28, A61K 9/16, A61K 31/343

(54) **COMPOSITE COMPRISING AMORPHOUS SOLID DISPERSION**

(30) Priority: 15.04.2024 JP 2024065705
(71) Applicant: SHIN-ETSU CHEMICAL CO., LTD., Tokyo 1000005 (JP)
(72) Inventor: ISHIMARU, Mitsuo, Kanagawa, 240-0005 (JP); WARASHINA, Shogo, Kanagawa, 240-0005 (JP); HOSHINO, Takafumi, Kanagawa, 240-0005 (JP)
(74) Representative: De Vries & Metman

(57) **Abstract**

There is provided a composite having a higher dissolution of a drug, the composite including at least a solid dispersion containing the drug and a carrier other than polyvinylpyrrolidone as well as HPMCAS outside the solid dispersion. More specifically, there is provided a composite including at least a solid dispersion containing at least a drug and a carrier selected from the group consisting of a vinylpyrrolidone-vinyl acetate copolymer, methyl cellulose, hydroxypropyl methyl cellulose, hydroxypropyl methyl cellulose phthalate and first hydroxypropyl methyl cellulose acetate succinate; and second hydroxypropyl methyl cellulose acetate succinate outside the solid dispersion.

## Description

### BACKGROUND OF THE INVENTION

### 1. Field of the Invention

The invention relates to a composite comprising a solid dispersion and hydroxypropyl methyl cellulose acetate succinate outside the solid dispersion.

### 2. Related Art

A solid dispersion can be produced, for example, by dissolving a drug and a carrier in a solvent and then removing the solvent for deposition (JP 2016-098179A). The solid dispersion, where a drug in an amorphous state is molecularly dispersed in the carrier, apparently and remarkably increases the solubility of the drug to improve bioavailability.

Although the solid dispersion in which a drug is dispersed in an amorphous state can improve the absorption of the drug after oral ingestion, there is a problem of physical stability, causing recrystallization of the drug during storage of the solid dispersion. In order to enhance the stability, a pharmaceutical composition containing a solid dispersion and a stabilizer outside the solid dispersion has been proposed, and experimental results of a pharmaceutical composition containing a solid dispersion containing polyvinylpyrrolidone (hereinafter also referred to as "PVP") as a carrier and hypromellose acetate succinate (another name: hydroxypropyl methyl cellulose acetate succinate, hereinafter also referred to as "HPMCAS") as an external stabilizer have been reported (JP 2023-515764A).

The same stabilizing effect was obtained even when hydroxypropyl methyl cellulose (hereinafter also referred to as "HPMC") was used in place of HPMCAS as the external stabilizer in the solid dispersion containing PVP as the carrier. For this reason, it is reported that the stabilization by HPMCAS is not caused by the intermolecular interaction between the acetate and succinate groups and the solid dispersion (European Journal of Pharmaceutics and Biopharmaceutics 2021,169,189-199).

### SUMMARY OF THE INVENTION

JP 2023-515764A describes "polyvinylpyrrolidone is most preferably used as a solid dispersion matrix agent.", and PVP was actually used as a carrier. However, it merely exemplifies a large number of carrier candidates other than PVP, so that the relation between a solid dispersion containing a carrier other than PVP and the external HPMCAS is unknown. European Journal of Pharmaceutics and Biopharmaceutics 2021,169,189-199 also describes use of PVP as a carrier, so that the results for the other carriers are unknown.

An object of the invention is to provide a pharmaceutical composition having higher dissolution of a drug, the composition comprising at least a solid dispersion comprising a carrier other than PVP, and HPMCAS outside the solid dispersion.

The inventors have found that the dissolution of a drug depends not only on the type of carrier in the solid dispersion, but also on the relation between the carrier and HPMCAS outside the solid dispersion. More particularly, the inventors have found that the combination of methyl cellulose and HPMCAS greatly improves the dissolution of a drug, although the methyl cellulose has hardly been used to date as a carrier of the solid dispersion. Consequently, the invention has been completed.

In one aspect of the invention, there is provided a composite comprising at least:
a solid dispersion comprising at least a drug and a carrier selected from the group consisting of a vinylpyrrolidone-vinyl acetate copolymer, methyl cellulose, hydroxypropyl methyl cellulose, hydroxypropyl methyl cellulose phthalate and first hydroxypropyl methyl cellulose acetate succinate; and
second hydroxypropyl methyl cellulose acetate succinate outside the solid dispersion.

In another aspect of the invention, there is provided a method of producing a composite comprising at least steps of:
preparing a solid dispersion comprising at least a drug and a carrier selected from the group consisting of a vinylpyrrolidone-vinyl acetate copolymer, methyl cellulose, hydroxypropyl methyl cellulose, hydroxypropyl methyl cellulose phthalate and first hydroxypropyl methyl cellulose acetate succinate; and
adding second hydroxypropyl methyl cellulose acetate succinate to the solid dispersion.

According to the invention, a composite having high dissolution including high initial dissolution may be provided.

### BRIEF EXPLANATION OF DRAWING

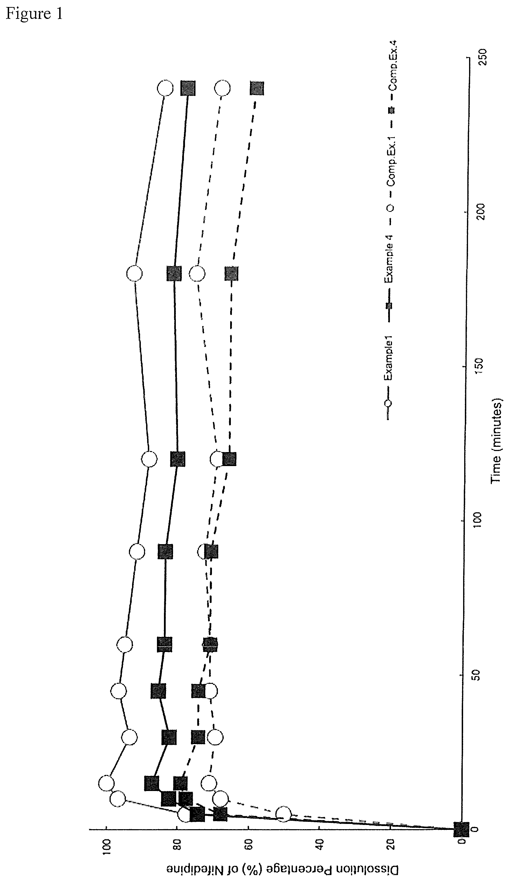

Figure 1 is a graph showing the relationship between the dissolution time and the dissolution percentage (%) of nifedipine in Examples 1 and 4 and Comparative Examples 1 and 4.

### DETAILED DESCRIPTION OF THE PREFERRED EMBODIMENTS

### (1) Solid dispersion

The solid dispersion comprises at least a drug and a carrier.

The drug contained in the solid dispersion is not particularly limited as long as it is orally administrable, and may be used singly or in combination of two or more. Examples of the drug include a drug for the central nervous system, a drug for the cardiovascular system, a drug for the respiratory system, a drug for the digestive system, an antibiotic, an antitussive and expectorant, an antihistamine, an antipyretic anti-inflammatory analgesic, a diuretic, an autonomic agent, an antimalarial agent, an antidiarrheal agent, a psychotropic, and vitamins and derivatives thereof.

Examples of the drug for the central nervous system include diazepam, idebenone, aspirin, ibuprofen, paracetamol, naproxen, piroxicam, diclofenac, indomethacin, sulindac, lorazepam, nitrazepam, phenytoin, acetaminophen, ethenzamide, ketoprofen, and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, propranolol hydrochloride, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as 2-[[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive and expectorant include noscapine hydrochloride, carbetapentane citrate, dextromethorphan hydrobromide, isoaminile citrate, and dimemorfan phosphate.

Examples of the antihistamine include chlorpheniramine maleate, diphenhydramine hydrochloride, and promethazine hydrochloride.

Examples of the antipyretic anti-inflammatory analgesic include ibuprofen, diclofenac sodium, flufenamic acid, sulpyrine, aspirin, and ketoprofen.

Examples of the diuretic include caffeine.

Examples of the autonomic agent include dihydrocodeine phosphate, dl-methylephedrine hydrochloride, propranolol hydrochloride, atropine sulfate, acetylcholine chloride, and neostigmine.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, calcium pantothenate, and tranexamic acid.

The solid dispersion can improve the solubility of the poorly water-soluble drug. Poorly water-soluble drugs refer to drugs listed in the Japanese Pharmacopoeia Eighteenth Edition as "slightly soluble", "very slightly soluble", or "practically insoluble or insoluble" in water. The term "slightly soluble" means that 1 g or 1 mL of a solid pharmaceutical product dissolves in 100 mL or more and less than 1000 mL of water within 30 minutes when placed in a beaker and shaken vigorously at 20 ± 5°C for 30 seconds every 5 minutes. The term "very slightly soluble" means that 1 g or 1 mL of a solid pharmaceutical product dissolves in 1000 mL or more and less than 10000 mL of water within 30 minutes in the same manner. The term "practically insoluble or insoluble" means that 1 g or 1 mL of a solid pharmaceutical product dissolves in 10000 mL or more of water within 30 minutes in the same manner.

In the above test of pharmaceutical product, dissolution of a poorly water-soluble drug means that the drug dissolves or becomes miscible in water, and also means that fibers, etc. are found to be un-present or present at a very slight amount.

Examples of the poorly water-soluble drug include azole-based compounds such as itraconazole, ketoconazole, fluconazole, miconazole and posaconazole; dihydropyridine-based compounds such as nifedipine, nitrendipine, amlodipine, nicardipine, nilvadipine, ferrodipine and efonidipine; propionic acid-based compounds such as ibuprofen, ketoprofen and naproxen; indoleacetic acid-based compounds such as indomethacin and acemetacin; griseofulvin; phenytoin; carbamazepine; dipyridamole; apalutamide; telaprevir; vemurafenib; ivacaftor; lumacaftor; tezacaftor; elexacaftor; enasidenib; doravirine; enzalutamide; ivosidenib; ibrutinib; deucravacitinib; and pirtobrutinib.

The carrier contained in the solid-dispersion is selected from the group consisting of a vinylpyrrolidone-vinyl acetate (VP/VA) copolymer, methyl cellulose, hydroxypropyl methyl cellulose (hereinafter also referred to as "HPMC"), hydroxypropyl methyl cellulose phthalate (hereinafter also referred to as "HPMCP") and hypromellose acetate succinate (hereinafter also referred to as "HPMCAS").

The vinylpyrrolidone-vinyl acetate (VP/VA) copolymer is a copolymer having a molar ratio of vinyl pyrrolidone (VP) monomer units to vinyl acetate (VA) monomer units of preferably from 5:5 to 7:3, more preferably 6:4. Examples of the VP/VA copolymer include Kollidon^{®} VA64 produced by BASF Co., which is a vinylpyrrolidone-vinyl acetate random copolymer having a molar ratio of vinyl pyrrolidone units to vinyl acetate units of 6:4.

Regarding the methyl cellulose, the degree of substitution (DS) of the methoxy groups is preferably from 1.54 to 2.03, and more preferably from 1.64 to 2.03. The DS means the degree of substitution and is the number of alkoxy groups per anhydroglucose unit (AGU) of cellulose. The DS of methoxy groups of methyl cellulose can be determined by the calculation based on the values obtained by the measurements in accordance with the Japanese Pharmacopoeia Eighteenth Edition.

Methyl cellulose having a wide range of viscosity may be used. It has surprisingly been found that methyl cellulose having a particularly low viscosity may contribute to the higher dissolution of a drug. The viscosity at 20°C of the 2% by mass aqueous solution of the methyl cellulose with an Ubbelohde-type viscometer is preferably from 1.0 to 50.0 mPa·s, more preferably 1.0 to 20.0 mPa·s, and still more preferably from 2.0 to 8.0 mPa·s.

When the viscosity at 20°C of a 2% by mass aqueous solution of the methyl cellulose is 600 mPa·s or more, it may be measured using a single cylinder-type rotational viscometer in accordance with the viscosity measurement by rotational viscometer in the Viscosity Determination of the General Tests in the Japanese Pharmacopoeia Eighteenth Edition. When the viscosity is less than 600 mPa·s, it may be measured using a Ubbelohde-type viscometer in accordance with the viscosity measurement by capillary tube viscometer in the Viscosity Determination of the General Tests in the Japanese Pharmacopoeia Eighteenth Edition.

Regarding HPMC, the degree of substitution (DS) of the methoxy groups is not particularly limited, and is preferably from 1.0 to 2.2, more preferably from 1.5 to 2.2, and still more preferably from 1.7 to 2.2. By using this degree of substitution (DS), preferable solubility in organic solvents may be ensured. The degree of substitution (DS) of methoxy groups mean an average number of methoxy groups per anhydroglucose unit (AGU).

The molar substitution (MS) of the hydroxypropoxy groups of the HPMC is not particularly limited, and is preferably from 0.10 to 1.00, more preferably from 0.20 to 0.80, and still more preferably from 0.20 to 0.65. By using this molar substitution (MS), preferable solubility in organic solvents may be ensured. The molar substitution (MS) of hydroxypropoxy groups means an average number of moles of hydroxypropoxy groups per mole of anhydroglucose unit (AGU).

The DS of methoxy groups and the MS of hydroxypropoxy groups of HPMC may be calculated based on the values obtained by the measurements in accordance with the Japanese Pharmacopoeia Eighteenth Edition.

The HPMC is preferably of type 2910 (methoxy group content of from 28.0 to30.0%, hydroxypropoxy group content of from 7.0 to 12.0%), type 2906 (methoxy group content of from 27.0 to 30.0%, hydroxypropoxy group content of from 4.0 to 7.5%), or type 2208 (methoxy group content of from 19.0 to 24.0%, hydroxypropoxy group content of form 4.0 to 12.0%), each described in the Official Monograph "Hypromellose" of the Japanese Pharmacopoeia Eighteenth Edition; more preferably of type 2910 or type 2906; and particularly preferably of type 2910. By using this HPMC, preferable solubility in organic solvents may be ensured.

The viscosity at 20°C of the 2% by mass aqueous solution of the HPMC is not particularly limited, and is preferably from 1.0 to 50 mPa·s, more preferably from 1.0 to 20mPa·s, and still more preferably from 2.0 to 8.0 mPa·s.

When the viscosity at 20°C of a 2% by mass aqueous solution of the HPMC is 600 mPa·s or more, it may be measured using a single cylinder-type rotational viscometer in accordance with the viscosity measurement by rotational viscometer in the Viscosity Determination of the General Tests in the Japanese Pharmacopoeia Eighteenth Edition. When the viscosity is less than 600 mPa·s, it may be measured using an Ubbelohde-type viscometer in accordance with the viscosity measurement by capillary tube viscometer in the Viscosity Determination of the General Tests in the Japanese Pharmacopoeia Eighteenth Edition.

Regarding the HPMCP, the degree of substitution (DS) of the methoxy groups is not particularly limited, and is preferably from 1.10 to 2.20, more preferably from 1.30 to 2.10, still more preferably from 1.60 to 2.00, and most preferably from 1.80 to 2.00. The molar substitution (MS) of the hydroxypropoxy groups is not particularly limited, and is preferably from 0.10 to 1.00, more preferably from 0.10 to 0.80, still more preferably from 0.15 to 0.60, and most preferably from 0.20 to 0.50. The degree of substitution (DS) of the carboxybenzoyl groups of the HPMCP is preferably from 0.10 to 2.50, more preferably from 0.10 to 1.00, and still more preferably from 0.40 to 0.80.

The DS of the methoxy groups, the DS of the carboxybenzoyl groups and the MS of the hydroxypropoxy groups of the HPMCP may be calculated based on the values obtained by the methods described in the Official Monographs "Hypromellose" and "Hypromellose Phthalate" in the Japanese Pharmacopoeia Eighteenth Edition. The degree of substitution (DS) of the methoxy groups and the DS (degree of substitution) of the carboxybenzoyl groups of the of HPMCP mean an average number of methoxy groups per anhydroglucose unit (AGU) and an average number of carboxybenzoyl groups per anhydroglucose unit (AGU), respectively. The molar substitution (MS) of hydroxypropoxy groups of the HPMCP means the average number of moles of hydroxypropoxy groups per anhydroglucose unit (AGU).

The viscosity at 20°C of a 10% by mass HPMCP solution in which the HPMCP is dissolved in a mixture of methanol and methylene chloride at a mass ratio of 1:1 is not particularly limited, and is preferably from 10.0 to 300.0 mPa·s, more preferably from 15.0 to 250.0 mPa·s, and still more preferably from 15.0 to 220.0 mPa·s.The viscosity at 20°C of the 10% by mass HPMCP solution in which the HPMCP is dissolved in a mixture of methanol and methylene chloride at a mass ratio of 1:1 may be measured using an Ubbelohde viscometer in accordance with the method described in the Official Monograph "Hypromellose phthalate" of the Japanese Pharmacopoeia Eighteenth Edition.

HPMCAS to be used as the carrier is not necessary to have the same degree of substitution, the same molar substitution, the same viscosity or the like as that of HPMCAS outside the solid dispersion. However, it may be in the same range of degree of substitution, the same molar substitution, the same viscosity or the like as the range of that of the below-described HPMCAS outside the solid dispersion.

The amount of the drug is preferably from 10 to 100 parts by mass, more preferably from 15 to 80 parts by mass, and still more preferably from 15 to 50 parts by mass, relative to 100 parts by mass of the carrier selected from the group consisting of a vinylpyrrolidone-vinyl acetate (VP/VA) copolymer, methyl cellulose, HPMC, HPMCP and HPMCAS. This is because the carrier in the solid dispersion and the hydroxypropyl methyl cellulose acetate succinate outside the solid dispersion provide a preferably high storage stability of the amorphous state of drug.

The solid dispersion may be prepared by, for example, a spray drying method in which a drug (preferably a poorly water-soluble drug) and a carrier are dissolved in a solvent and then spray-dried, or a hot-melt extrusion method in which the drug and a polymer are thermally melted and extruded. The solid dispersion to be used for the invention may be any solid dispersion prepared by any method.

For example, the spray drying method includes a broad range of method in which a spray dry stock solution containing a drug and a polymer serving as a carrier together with a solvent is dispersed (sprayed) into small droplets, and the solvent is rapidly removed from the droplets by evaporation. The driving force for removing the solvent is generally obtained by lowering the partial pressure of the stock solution relative to the vapor pressure of the solvent at a temperature at which the droplets are dried. Preferred embodiments include keeping the pressure in the solvent removal vessel in an incomplete vacuum, when the droplets are mixed with the hot drying gas in the solvent removal vessel.

The solvent may be any solvent as long as it is capable of dissolving the drug, the carrier, and an optional additive described below. Examples of the preferable solvent include water, acetone, methanol, ethanol, isopropanol, methyl acetate, ethyl acetate, tetrahydrofuran and dichloromethane. The solvent may be used singly or as a mixture of two or more solvents. When the solution of the solid dispersion comprises a water-miscible solvent, water can be added to the solution of the solid dispersion.

The spray dry stock solution containing the drug and the carrier together with the solvent may be spray-dried under various nozzle mechanisms. For example, various types of nozzles may be used. Examples of preferable nozzles include two-fluid nozzles, fountain nozzles, flat fan nozzles, pressure nozzles, and rotary atomizers.

The spray dry stock solutions may be fed at wide ranges of flow rates and temperatures. When pressurized during spraying, it is possible to spray at a wide range of pressure. In general, as the specific surface area of the droplet increases, the rate of solvent evaporation increases. For this reason, the size of the droplet coming out of the nozzle is preferably less than 500 µm, more preferably less than 400 µm, and still more preferably from 5 to 200 µm. The flow rate, temperature and pressure are preferably selected to allow said size of the droplet to be obtained. After sprayed, the stock solution rapidly solidifies.

After sprayed, the spray dry stock solution rapidly solidifies into a solid dispersion. The solidified solid dispersion generally remains in the spray drying chamber for about 5 seconds to about 60 seconds during which the solvent is removed from the solid powder. Regarding the temperature during spray drying, the inlet temperature is preferably from about 20°C to about 150°C, and the outlet temperature is from about 0°C. to about 85°C.

A smaller residual solvent in the solid dispersion is preferable. This is because the mobility of the drug molecules in the amorphous solid dispersion is suppressed to increase the stability. Secondary drying may be carried out when further removal of the residual solvent is necessary. Examples of the preferable secondary drying method include tray drying, fluidized bed drying, belt drying, and microwave drying.

The solid dispersion may contain a various optional additive which may be commonly used in the art. Examples of the optional additive include an excipient, a binder, a disintegrant, a lubricant and an anti-agglomerating agent.

Examples of the excipient include saccharides such as sucrose, lactose, mannitol and glucose; starch, and microcrystalline cellulose.

Examples of the binder include polyvinyl alcohol, polyacrylic acid, polyvinylpyrrolidone, hydroxyethyl cellulose, hydroxypropyl methyl cellulose cellulose, hydroxypropyl cellulose, macrogols, gum arabic, gelatin and starch.

Examples of the disintegrant include low-substituted hydroxypropyl cellulose, carmellose or a salt thereof, croscarmellose sodium, sodium carboxymethyl starch, crospovidone, microcrystalline cellulose, and microcrystalline cellulose-carmellose sodium.

Examples of the lubricant and the anti-aggregation agent include talc, magnesium stearate, calcium stearate, silicon dioxide (colloidal silica), stearic acid, waxes, hydrogenated oils, polyethylene glycols and sodium benzoate.

The hot melt extrusion method is a method in which an amorphous solid dispersion is obtained by kneading and extruding raw material at a temperature equal to or higher than a glass transition temperature of raw material. The hot melt extrusion may be carried out using a hot melt extruder. The hot melt extruder is not particularly limited as long as it is an extruder having a structure in which a polymer to be used as a carrier, an active ingredient, a plasticizer and a surfactant are thermally melted and kneaded while applying a shearing force to them with a piston or a screw, and then extruded from a die. A twin screw extruder is preferable. By using the twin extruder, preferably more uniform extrudate is obtained.

A hot melt extrudate may be obtained by selecting the shape of the die and extruding into a desired shape such as a circular shape or a quadrilateral shape as well as a columnar shape or a film shape.

Specific examples of the hot melt extruder include a capillary graph (single screw piston-type extruder) produced by Toyo Seiki Seisaku-sho, Ltd., Nano-16 (twin screw-type extruder) produced by Leistritz Corporation, Process11 (twin screw-type extruder) produced by Thermo Fischer Scientific K. K., and Pharma11 (twin screw-type extruder).

The hot-melt temperature is preferably from 50 to 200°C, more preferably from 60 to 180°C, still more preferably from 80 to 160°C, and particularly preferably from 80 to 150°C. By using this hot-melt temperature, preferable stability of the drug (preferably a poorly water-soluble drug) and the carrier may be ensured.

The conditions for the hot melt extrusion are not particularly limited. When a single screw piston-type extruder is used, the extrusion speed is preferably from 1 to 1000 mm/ minutes, more preferably from 10 to 500 mm/ minutes. When a twin screw-type extruder is used, the screw revolutions are preferably from 1 to 1000 rpm, more preferably from 1 to 500 rpm.

The hot-melt extrudate is cooled after coming out of the die, by natural cooling at room temperature (25 to 30°C) or by cold blowing.

The cooled hot-melt extrudate may be subjected to optional pelletization with a cutting machine to obtain pellets having sizes of from 0.1 to 5 mm, and further subjected to optional pulverization for controlling particle sizes until obtaining granulates or a powder.

The cutting machine is preferably a pelletizer, a knife mill, or the like, which is capable of easy pelletization. By using this cutting machine, preferable pulverization of the extrudate may be ensured.

The pulverizer is preferably a jet mill, a knife mill, a pin mill, or the like, which hardly increases the product temperature due to the apparatus structure.

When the inside temperatures of the cutting machine and the pulverizer become high, thermally softened particles adhere to each other so that the pulverization is preferably carried out under cooling by blowing or the like.

### (2) Later Addition of HPMCAS

The dissolution of the produced solid dispersion can be improved by placing HPMCAS outside the solid dispersion.

The HPMCAS may be produced using, for example, the method described in JPS54-61282A. Hypromellose (another name: hydroxypropyl methyl cellulose, hereinafter also referred to as "HPMC") as a starting material is dissolved in glacial acetic acid; then subjected to addition of acetic anhydride and succinic anhydride as esterifying agents, and sodium acetate as a reaction catalyst; and the resulting mixture is heated for the esterification reaction. After completion of the reaction, a large amount of water is added to the reaction mixture to allow HPMCAS to precipitate, and the precipitate is washed with water and dried.

The degree of substitution (DS) of the methoxy groups of HPMCAS is preferably from 1.10 to 2.20, more preferably from 1.40 to 2.00, and still more preferably from 1.60 to 2.00.

The molar substitution (MS) of the hydroxypropoxy groups of HPMCAS is preferably from 0.10 to 1.00, more preferably from 0.20 to 0.80, and still more preferably from 0.20 to 0.65.

The degree of substitution (DS) of the acetyl groups of HPMCAS is preferably from 0.10 to 2.50, more preferably from 0.10 to 1.00, and still more preferably from 0.20 to 0.80.

The degree of substitution (DS) of the succinyl groups of HPMCAS is preferably from 0.10 to 2.50, more preferably from 0.10 to 1.00, and still more preferably from 0.10 to 0.60.

The DS or MS of methoxy groups, hydroxypropoxy groups, acety groups and succinyl groups of HPMCAS may be calculated based on the values obtained by the method described in the Official Monograph "Hypromellose Acetate Succinate" in the Japanese Pharmacopoeia Eighteenth Edition.

The volume-average particle size of HPMCAS may be measured with a dry laser diffraction particle size analyzer. The volume-average particle size is calculated using the equation {Σ(nD³)/Σn}^{1/3} as described, for example, in "Revised and Enlarged Edition Funtai Bussei Zusetsu (Powder Property Diagram)" by the Society of Powder Technology, Japan, and the Association of Powder Process Industry and Engineering, Japan; Nikkei Technical Book, 1985, page 88. In the equation, "D" denotes a diameter (or size) of particle, "n" denotes the number of particles having said diameter, and "Σn" denotes the total number of particles.

The dry laser diffraction particle size analyzer is a device in which a powder sample is ejected with compressed air, and subjected to irradiation with a laser beam to measure the volume-average particle size based on the diffraction intensity. Examples of the dry laser diffraction particle size analyzer include the Mastersizer produced by Malvern, UK, and the HELOS apparatus produced by Sympatec, Germany.

Regarding the volume particle size, D₅₀ means a cumulative particle size of 50%.

When HPMCAS powder is used for physical mixing, granulation or the like as the below-described addition to the solid dispersion, the volume-average particle size (D₅₀) of the HPMCAS powder is not particularly limited, and is preferably 2 mm or less, more preferably from 1 to 500 µm, and still more preferably from 2 to 300 µm. By using this volume-average particle size, preferably uniform mixing with the solid dispersion may be ensured.

The viscosity at 20°C of a 2% by mass HPMCAS solution in which the HPMCAS is dissolved in a dilute (0.1mol/L) aqueous sodium hydroxide solution is preferably from 1.1 to 20 mPa·s, and more preferably from 1.5 to 3.6 mPa·s. When the viscosity is less than 1.1 mPa·s, the mist may become finer during spraying and cannot be recovered. When the viscosity is more than 20 mPa·s, the viscosity of liquid composition is increased so that the productivity during spray drying is significantly reduced. The viscosity may be measured by the method described in the General Tests and Official Monograph "HPMCAS" in the Japanese Pharmacopoeia Eighteenth Edition.

The HPMCAS outside the solid dispersion is preferably not greater than the amount of carrier present in the solid dispersion, and is preferably from 10 to 100 parts by mass, more preferably from 10 to 90 parts by mass, and still more preferably from 20 to 85 parts by mass, relative to 100 parts by mass of the carrier. It is because the carrier in the solid dispersion and the hydroxypropyl methyl cellulose acetate succinate outside the solid dispersion provide a preferably high storage stability of the amorphous state of drug.

Relative to 100 parts by mass of the carrier, it is preferable to have 10 to 100 parts by mass of the drug and 10 to 100 parts by mass of the HPMCAS outside the solid dispersion, and it is more preferable to have 15 to 80 parts by mass of the drug and 10 to 90 parts by mass of the HPMCAS outside the solid dispersion, and it is still more preferable to have 15 to 50 parts by mass of the drug and 20 to 85 parts by mass of the HPMCAS outside the solid dispersion. It is because the carrier in the solid dispersion and the hydroxypropyl methyl cellulose acetate succinate outside the solid dispersion provides a preferably high storage stability of the amorphous state of drug.

The solid dispersion and the HPMCAS may be combined by physical mixing, granulation or coating to form a composite (composition) containing the solid dispersion and the HPMCAS outside the solid dispersion.

Examples of the physical mixing include physically mixing at least the solid-dispersion and the HPMCAS in a mortar or the like, manually mixing them in a bag, and rotating a mixing vessel containing them in powder form for mixing them. Examples of the mixer with the mixing vessel include a V-type mixer with a V-shaped vessel, a ribbon-type mixer, a container-type mixer, and a tumbler-type mixer. At this time, a various additive which may be commonly used in this field may be added. Examples of the additive include an excipient, a binder, a disintegrant, a lubricant, and an anti-aggregation agent. Specific examples of the additive are the same as those described above for the solid dispersion.

Examples of the granulation method include, but are not limited to, a dry granulation method and a wet granulation method. The granulation method may be performed using a commonly used granulator.

Examples of the granulator include, but are not limited to, a dry granulator such as a roller compactor; and a wet granulator such as a high-speed stirring granulator, a fluidized bed granulator, a tumbling fluidized bed granulator and a spray-dry granulator. The mixing time or the granulation time is not particularly limited, and is typically from 1 to 120 minutes.

For example, in the dry granulation in which roller compaction is performed by using, for example, a compaction granulator such as a roller compactor, a various additive which may be commonly used in this field may be added in addition to the solid dispersion and the HPMCAS. Examples of the additive include an excipient, a binder, a disintegrant, a lubricant and an anti-agglomeration agent. Examples of the additive are the same as those described above for the solid dispersion.

The roll pressure varies depending on the powder properties or the like, and is preferably from 1 to 30 MPa, more preferably from 2 to 12 MPa, and the roll rotation speed is preferably from 1 to 50 rpm, more preferably from 2 to 20 rpm. The screw rotation speed is preferably from 1 to 100 rpm, and more preferably from 2 to 50 rpm. The flakes of the compressed granulated product, obtained by the roller compaction, may be pulverized and sorted with a pulverizer or crusher such as a comill, a quick mill, a power mill, Granumeist^{®} or a roll granulator to obtain tableting powder having a predetermined particle size. The HPMCAS may be added during the granulation or after the granulation.

In the wet granulation, the HPMCAS may be contained in the powder to be granulated, or the HPMCAS dispersed or dissolved in the granulation liquid may be added. The granulation may be carried out by using a granulator. For example, when granulation is carried out using a fluidized bed granulator, the intake air temperature is preferably from 50 to 100°C and/or the exhaust gas temperature is preferably from 25 to 80°C. By using this intake air temperature and/or this exhaust gas temperature, preferable granulation progress efficiency and preferable quality of the granulation product may be ensured.

The average particle size of the granulation product is preferably from 50 to 500 µm, and more preferably from 150 to 250 µm. By using this average particle size, preferable subsequent tableting or capsule filling may be ensured. The average particle size of the granulation product is a value measured by a laser diffraction method or the like (50% cumulative value of the volume-based cumulative particle size distribution curve). Purified water, an organic solvent such as ethanol, or a mixed solution thereof may be typically used as the granulation liquid.

When a granulator (e.g., a fluidized bed granulator) capable of simultaneously performing spraying and drying is used, the obtained granulate is not required to be further dried. When drying is not performed or when a granulator (e.g., a wet stirring granulator) not capable of performing drying is used, drying is preferably performed by a known method. The drying may be done by using a dryer (including a drying oven). Examples of the dryer include a fluidized bed dryer, a flash dryer, a box type dryer, a vibration dryer, a natural convection type constant temperature dryer, a forced convection type constant temperature dryer, and a forced convection type constant temperature and constant humidity dryer. The drying temperature is preferably from 40 to 120°C.

The water content of the dried granulation product is preferably 5.0% by mass or less, more preferably 2.0% by mass or less. By using this water content, preferable tablet stability may be ensured. The water content of the granulation product may be determined by using a heating and drying method moisture analyzer (MX-50 produced by A&D Company Ltd.) under the conditions of 5 g of granulation product, a heating temperature of 105°C and a heating time of 60 minutes. The HPMCAS may be added during the granulation or after the granulation.

When the HPMCAS is added to the solid dispersion powder by physical mixing, the resulting physical mixture is subjected to tableting to obtain tablets. When the HPMCAS is added to the solid dispersion powder by granulation, the granules after the granulation are tableted to obtain tablets.

The tableting may be carried out by using a tableting machine. Examples of the tableting machine include a rotary tableting machine and a single punch tableting machine. The tableting pressure during tableting is preferably from 2 to 40 kN. By using this tableting pressure, preferable tablet hardness and less tableting failure may be ensured. The tablet design such as tablet size and tablet weight may be appropriately selected. For example, the tablet diameter is preferably from 6 to 12 mm. By using this tablet diameter, preferable handleability and easy dosage may be ensured. The mass of the tablet is not particularly limited, and is preferably from 70 to 700 mg per tablet.

When the tablet or granule contains, for example, a solid dispersion in the absence of HPMCAS outside the solid dispersion, HPMCAS can become present outside the solid dispersion by coating the tablet or granule with an enteric coating composition containing the HPMCAS. Even when the physically mixed powder, granulates, tablets or the like contains the solid-dispersion and HPMCAS outside the solid dispersion by the above-described method, it may be subjected to coating with an enteric coating composition containing HPMCAS. When HPMCAS is present in the solid dispersion and/or present outside the solid dispersion, HPMCAS to be used for the coating is not necessary to have the same degree of substitution (DS) or molar substitution (MS), viscosity or the like as that of the existing HPMCAS. Examples of the HPMCAS to be used for the coating may include the examples of HPMCAS present outside the solid dispersion described above. HPMCAS-coated granules may be further tableted using the method described above.

The enteric coating composition containing HPMCAS may further contain a optional additive in an amount commonly used in this field. Examples of the optional additive include a plasticize, a neutralizer, a lubricant, the other coating base, a surfactant, a sweetener, a pigment, and an antifoaming agent.

The coating may be carried out by a known method such as spray coating with a coating apparatus. Examples of the coating apparatus include a pan coating apparatus, a drum type coating apparatus, a fluidized bed coating apparatus, a stirred flow coating apparatus, and a rolling flow coating apparatus. Any of air spray, airless spray, three-fluid spray and the like may be used as a spray accompanied by the coating apparatus. The product temperature during coating is not particularly limited as long as the sprayed coating composition can be continuously dried, and is preferably from 20 to 80°C, and more preferably from 25 to 60°C.

The drying method is not particularly limited as long as the solvent can be removed. The coated product may be, for example, heated for drying in the coating apparatus or outside the coating apparatus after taken out of the coating apparatus. The blowing temperature used for drying is preferably from 25 to 90°C. By using this blowing temperature, drying the coating layer at a preferably sufficient speed while keeping the drug-containing core in a preferably desired level of moisture may be ensured.

In the case of a solid dispersion in a metastable state, dry coating without using a solvent is also one of effective coating methods. In the drying coating using HPMCAS, there is also used, for example, a plasticizer, an anti-adhesion agent or a wetting agent for promoting formation of film. Examples of the coating apparatus include a centrifugal rolling coating apparatus, a pan coating apparatus and a fluidized bed coating apparatus, thereby allowing the coating composition to be continuously dispersed. The centrifugal rolling coating apparatus is preferable. By using the centrifugal rolling coating apparatus, preferable stirring may be ensured. The temperature of the granules and tablets to be coated in the dry coating is preferably from 30°C or higher, more preferably from 40 to 80°C, and still more preferably from 50 to 80°C.

### EXAMPLES

The invention will be described with reference to Examples and Comparative Examples below. It should not be construed that the invention is limited to or by them.

### Example 1 (Mixture of methylcellulose to drug of 100:20, subjected to addition of 25 of HPMCAS-1)

### <Production of solid dispersion>

The 63 parts by mass of acetone was stirred with a stirring blade, while 1.67 parts by mass of nifedipine (produced by Daito Pharmaceutical Co., Ltd.) was added thereto for dissolving the nifedipine in the acetone. Then 8.33 parts by mass of methyl cellulose (DS of methoxy groups of 1.77, and viscosity at 20°C of 4.04 mPa·s determined in a 2% by mass aqueous solution) was added to the nifedipine solution and dispersed therein, followed by addition of 27 parts by mass of purified water thereto for completely dissolving the mehtyl cellulose to obtain a spray dry stock solution.

The prepared spray dry stock solution was spray-dried using a mini spray dryer B-290 (produced by Nihon BUCHI K. K.) under the conditions of an inlet temperature of 90°C, an outlet temperature of 50°C, a nozzle gas flow of 473 L/hr and a spray rate of 5.3 g/min to produce a solid dispersion.

### <Production of composite>

The 73.8 parts by mass of the produced solid dispersion, 15.2 parts by mass of HPMCAS (DS of methoxy groups of 1.88, MS of hydroxypropoxy groups of 0.24, DS of acetyl groups of 0.54, DS of succinyl groups of 0.28 and viscosity at 20°C of 2.87 mPas as determined in 2% by mass HPMCAS solution in which the HPMCAS is dissolved in a dilute (0.1 mol/L) sodium hydroxide aqueous solution, hereinafter also referred to as "HPMCAS-1"), 0.5 parts by mass of Adsolider^{®} 101 (silicon dioxide, produced by Freund Corporation), 10 parts by mass of croscarmellos sodium (Ac-Di-Sol SD-711 produced by Signet Excipients) and 0.5 parts by mass of magnesium stearate (produced by Taihei Chemical Industrial Co., Ltd.) were thoroughly mixed in a mortar to produce a composite containing the solid dispersion and the HPMCAS outside the solid dispersion.

### <Dissolution test>

The produced composites were weighed in such an amount as to have a nifedipine content of 40 mg (44.4 mg/L), and subjected to a 240 minute dissolution test. The 2nd fluid (pH value of 6.8, 900 mL) described in the Disintegration Test of the Japanese Pharmacopeia Eighteenth Edition was used as the test liquid, and a dissolution test machine (NTR-6100A produced by Toyama Sangyo Co., Ltd.) was used. Since the solid dispersion is very fine and easily aggregated, the paddle rotation speed and paddle position were fixed to the conditions in Table 1 and evaluation was carried out.

**Table 1**

| test time | rotation speed of paddle (rpm) | position of paddle |
|---|---|---|
| from 0 to 10 minutes | 200 | 3.5 cm below the liquid surface |
| more than 10 minutes and 240 minutes or less | 100 | 10 cm below the liquid surface |

The dissolution amount of nifedipine was calculated from the absorbance obtained by UV spectrum (wavelength of 325 nm and optical path length of 10 mm) based on a concentration conversion line (calibration curve) prepared at known concentrations. The results are shown in Table 2.

### Example 2 (Mixture of HPMC to drug of 100:33, subjected to addition of 67 of HPMCAS-1)

### <Production of solid dispersion>

The 72 parts by mass of ethanol was stirred with a stirring blade, while 2.5 parts by mass of nifedipine was added thereto for dissolving the nifedipine in the ethanol. Next, 7.5 parts by mass of hypromellose (HPMC) (DS of methoxy groups of 1.89, MS of hydroxypropoxy groups of 0.24 and viscosity at 20°C of 3.04 mPa·s as determined in a 2% by mass aqueous solution) was added to the nifedipine solution and dispersed therein, followed by addition of 18 parts by mass of purified water thereto for completely dissolving the HPMC to obtain a spray dry stock solution.

The prepared spray dry stock solution was spray-dried using a min spray dryer B-290 (produced by Nihon BUCHI K.K.) under the conditions of an inlet temperature of 120°C, an outlet temperature of 68°C, a nozzle gas flow of 357 L/hr and a spray rate of 6.5 g/min to produce a solid dispersion.

### <Production of composite>

The 49.2 parts by mass of the produced solid dispersion, 24.6 parts by mass of HPMCAS-1, 15.2 parts by mass of microcrystalline cellulose, 0.5 parts by mass of Adsolider^{®} 101, 10% by mass of croscarmellose sodium and 0.5 parts by mass of magnesium stearate were thoroughly mixed in a mortar to produce a composite containing the solid dispersion and the HPMCAS outside the solid dispersion.

### <Dissolution test>

The dissolution test was carried out in the same manner as in Example 1 except that the produced composite was weighed in such an amount as to have the nifedipine content of 80 mg (88.8 mg/L). The results are shown in Table 2.

### Example 3 (Mixture of HPMCP to drug of 100:33, subjected to addition of 67 of HPMCAS-1)

### <Production of solid dispersion>

The 90 parts by mass of acetone was stirred with a stirring blade, while adding 2.5 parts by mass of nifedipine thereto for dissolving the nifedipine in the acetone. Next, 7.5 parts by mass of hypromellose phthalate (HPMCP) (DS of methoxy groups of 1.89, MS of hydroxypropoxygroups of 0.25, DS of carboxybenzoyl groups of 0.67 and viscosity at 20°C of 43.2 mPa·s as determined in a 10% by mass HPMCP solution in which the HPMCP is dissolved in a mixed solvent of methanol and dichloromethane at a mass ratio 1:1) was added to the nifedipine solution and completely dissolved therein to obtain a spray dry stock solution.

The prepared spray dry stock solution was spray-dried using a mini spray dryer B-290 (Nihon BUCHI K.K.) under the conditions of an inlet temperature of 80°C, an outlet temperature of 50°C, a nozzle gas flow of 357 L/hr and a spray rate of 4.4 g/min to produce a solid dispersion.

### <Production of Composite>

A composite containing the solid dispersion and HPMCAS outside the solid dispersion was produced in the same manner as in Example 2.

### <Dissolution test>

The dissolution test was carried out in the same manner as in Example 1. The results are shown in Table 2.

### Example 4 (Mixture of VP/VA copolymer to drug of100:20, subjected to addition of 25 of HPMCAS-1)

A spray dry stock solution, a solid dispersion and a composite were produced in the same manner as in Example 1 except that a vinylpyrrolidone-vinyl acetate copolymer (VP/VA copolymer) (Kollidon^{®} VA64 produced by BASF Corporation) was used instead of the methyl cellulose, and the dissolution test was carried out. The results are shown in Table 2.

### Example 5 (Mixture of methyl cellulose to drug of 100:25, subjected to addition of 50 of HPMCAS-1)

### <Production of solid dispersion>

The 63 parts by mass of acetone was stirred with a stirring blade, while 2.0 parts by mass of griseofulvin (produced by Tokyo Chemical Industry Co., Ltd.) was added thereto for dissolve the griseofulvin in the acetone. Then 8.0 parts by mass of methyl cellulose (DS of methoxy groups of 1.77, and viscosity at 20°C of 4.04 mPa·s determined in a 2% by mass aqueous solution) was added to the griseofulvin solution and dispersed therein, followed by addition of 27 parts by mass of purified water thereto for completely dissolving the methyl cellulose to obtain a spray dry stock solution.

The produced spray dry stock solution was spray-dried using a mini spray dryer B-290 (Nihon BUCHI K.K>) under the conditions of an inlet temperature of 90°C, an outlet temperature of 50°C, a nozzle gas flow of 473 L/hr and a spray rate of 5.5 g/min to obtain a solid dispersion.

### <Production of composite>

The 62.5 parts by mass of the produced solid dispersion, 24.6 parts by mass of HPMCAS-1, 2.9 parts by mass of microcrystalline cellulose, 0.5 parts by mass of Adsolider^{®} 101, 10 parts by mass of croscarmellose sodium and 0.5 parts by mass of magnesium stearate were thoroughly mixed in a mortar to produce a composite containing the solid dispersion and the HPMCAS outside the solid dispersion.

### <Dissolution test>

A dissolution test of the produced composite was carried out in the same manner as in Example 1 except that the content of griseofulvin was 50 mg (55.6 mg/L). The results are shown in Table 2.

### Examples 6 (Mixture of methylcellulose to drug of 100:25, subjected to addition of 50 of HPMCAS-1)

A spray dry stock solution, a solid dispersion and a composite were produced in the same manner as in Example 5 except that nifedipine was used instead of griseofulvin, and the dissolution test was carried out. The results are shown in Table 2.

### Example 7 (Mixture of methyl cellulose to drug of 100:33, subjected to addition of 67 of HPMCAS-1)

### <Production of solid dispersion>

The 63 parts by mass of acetone was stirred with a stirring blade, while adding 2.5 parts by mass of nifedipine thereto for dissolving the nifedipine in the acetone. Then 7.5 parts by mass of methyl cellulose (DS of methoxy groups of 1.77, and viscosity at 20°C of 4.04 mPa·s determined in a 2% by mass aqueous solution) was added to the nifedipine solution and dispersed therein, followed by addition of 27 parts by mass of purified water for completely dissolving the methyl cellulose to obtain a spray dry stock solution. The spray dry solution was spray-dried under the same conditions as in Example 1 to obtain a solid dispersion.

### <Production of composite>

The 49.2 parts by mass of the produced solid dispersion, 24.6 parts by mass of HPMCAS-1, 15.2 parts by mass of microcrystalline cellulose, 0.5 parts by mass of Adsolider^{®} 101, 10 parts by weight of croscarmellose sodium and 0.5 parts by mass of magnesium stearate were thoroughly mixed in a mortar to produce a composite containing the solid dispersion and HPMCAS outside the solid dispersion.

### <Dissolution test>

The dissolution test was carried out in the same manner as in Example 1. The results are shown in Table 2.

### Example 8 (Mixture of HPMCAS-2 to drug at 100:33, subjected to addition of 67 of HPMCAS-1)

### <Production of solid dispersion>

The 75 parts by mass of HPMCAS (DS of methoxy groups of 1.90, MS of hydrocypropxy groups of 0.24, DS of acetyl groups of 0.49, DS of succinyl groups of 0.38, a volume-average particle size D₅₀ of 278 µm and viscosity at 20°C of 3.08 mPa·s as determined in a 2% by mass HPMCAS solution in which the HPMCAS was dissolved in a dilute (0.1 mol/L) NaOH solution, hereinafter also referred to as "HPMCAS-2") and 25 parts by mass of nifedipine were manually and thoroughly mixed in a bag, and fed into a Volumetric Feeder (produced by Thermo Fisher Scientific K.K.). The mixed powders of HPMCAS-2 and nifedipine were fed from the Volumetric Feeder into a hot-melt extruder at a rate of 5.6 g/mim to produce a solid dispersion. A Process11 (produced by Thermo Fisher Scientific K.K.) was used as the hot-melt extruder, and the barrel temperature of 160°C and the screw rotation speed of 200 rpm were employed. The obtained extrudate was pelletized with a VariCut pelletizer (produced by Thermo Fisher Scientific K.K.), and then pulverized with a GENA (produced by Nara Machinery Co., Ltd.) equipped with a mesh having openings of 0.3 mm to obtain solid dispersion powder.

### <Production of composite>

The 49.2 parts by mass of the produced solid dispersion powder, 24.6 parts by mass of HPMCAS-1, 15.2 parts by mass of microcrystalline cellulose, 0.5 parts by mass of Adsolider^{®} 101, 10 parts by mass of croscarmellose sodium and 0.5 parts by mass of magnesium stearate were thoroughly mixed in a mortar to produce a composite containing the solid dispersion and HPMCAS outside the solid dispersion.

### <Dissolution test>

The dissolution test was carried out in the same manner as in Example 2. The results are shown in Table 2.

### Comparative Example 1 (Mixture of methyl cellulose to drug of 100:20, subjected to addition of 25 of microcrystalline cellulose)

A composite containing the solid dispersion without HPMCAS outside the solid dispersion was produced by using the method for producing the composite in Example 1, while replacing HPMCAS-1 by microcrystalline cellulose, and the dissolution test was carried out in the same manner as in Example 1. The results are shown in Table 2.

### Comparative Example 2 (Mixture of HPMC to drug of 100:33, subjected to addition of 67 of microcrystalline cellulose)

A composite containing the solid dispersion without HPMCAS outside the solid dispersion was produced by using the method for producing the composite in Example 2, while replacing HPMCAS-1 by microcrystalline cellulose, and the dissolution test was carried out in the same manner as in Example 2. The results are shown in Table 2.

### Comparative Example 3 (Mixture of HPMCP to drug of 100:33, subjected to addition of 67 of microcrystalline cellulose)

A composite containing the solid dispersion without HPMCAS outside the solid dispersion was produced by using the method for producing the composite in Example 3, while replacing HPMCAS-1 by microrystalline cellulose, and the dissolution test was carried out in the same manner as in Example 3. The results are shown in Table 2.

### Comparative Example 4 (Mixture of VP/VA copolymer to drug of 100:20, subjected to addition of 25 of microcrystalline cellulose)

A composite containing the solid dispersion without HPMCAS outside the solid dispersion was produced by using the method for producing the composite in Example 4, while replacing HPMCAS-1 by microcrystalline cellulose, and the dissolution test was carried out in the same manner as in Example 4. The results are shown in Table 2.

### Comparative Example 5 (Mixture of methyl cellulose to drug of 100:25, subjected to addition of 50 of microcrystalline cellulose)

A composite containing the solid dispersion without HPMCAS outside the solid dispersion was produced by using the method for producing the the composite in Example 5, while replacing HPMCAS-1 by microcrystalline cellulose, and the dissolution test was carried out in the same manner as in Example 5. The results are shown in Table 2.

### Comparative Example 6 (Mixture of methyl cellulose to drug of 100:25, subjected to addition of 50 of microcrystalline cellulose)

A composite containing the solid dispersion without HPMCAS outside the solid dispersion was produced by using the method for producing the composite in Example 6, while replacing HPMCAS-1 by microcrystalline cellulose, and the dissolution test was carried out in the same manner as in Example 6. The results are shown in Table 2.

### Comparative Example 7 (Mixture of methyl cellulose to drug of 100:33, subjected to addition of 67 of microcrystalline cellulose)

A composite containing the solid dispersion without HPMCAS outside the solid dispersion was produced by using the method for producing the composite in Example 7, while replacing HPMCAS-1 by microcrystalline cellulose, and the dissolution test was carried out in the same manner as in Example 7. The results are shown in Table 2.

### Comparative Example 8 (Mixture of methyl cellulose to drug of 100:33, subjected to addition of 67 of HPMC)

A composite containing the solid dispersion without HPMCAS outside the solid dispersion was produced by using the method for producing the composite in Example 7, while replacing HPMCAS-1 by HPMC, and the dissolution test was carried out in the same manner as in Example 7. The results are shown in Table 2.

### Comparative Example 9 (Mixture of HPMCAS-2 to drug of 100:33, subjected to addition of 67 of microcrystalline cellulose)

A composite containing the solid dispersion without HPMCAS outside the solid dispersion was produced by using the method for producing the composite in Example 8, while replacing HPMCAS-1 by microcrystalline cellulose, and the dissolution test was carried out in the same manner as in Example 8. The results are shown in Table 2.

**Table 2**

| | eluted amount (mass%) after elapsed time | | | | | | | | | | |
|---|---|---|---|---|---|---|---|---|---|---|---|
| | 0 min | 5 min | 10 min | 15 min | 30 min | 45 min | 60 min | 90 min | 120 min | 180 min | 240 min |
| Example1 | 0.0 | 77.5 | 96.9 | 100.1 | 93.7 | 96.9 | 95.3 | 92.1 | 88.8 | 93.7 | 85.6 |
| Example2 | 0.0 | 41.6 | 55.5 | 60.1 | 63.2 | 66.3 | 64.7 | 63.2 | 60.1 | 60.1 | 57.0 |
| Example3 | 0.0 | 88.8 | 93.7 | 92.1 | 92.1 | 93.7 | 88.8 | 88.8 | 90.4 | 95.3 | 88.8 |
| Example4 | 0.0 | 74.3 | 82.4 | 87.2 | 82.4 | 85.6 | 84.0 | 84.0 | 80.8 | 82.4 | 79.1 |
| Example5 | 0.0 | 89.5 | 100.4 | 101.7 | 101.7 | 104.4 | 103.1 | 103.1 | 103.1 | 101.7 | 103.1 |
| Example6 | 0.0 | 80.8 | 94.9 | 95.7 | 95.4 | 95.0 | 92.5 | 90.1 | 88.7 | 86.5 | 82.7 |
| Example7 | 0.0 | 66.2 | 77.5 | 80.8 | 77.5 | 82.4 | 77.5 | 84.0 | 79.1 | 82.4 | 80.8 |
| Example8 | 0.0 | 69.4 | 89.4 | 94.0 | 91.0 | 92.5 | 89.4 | 87.9 | 86.3 | 75.5 | 61.7 |
| Comp.Ex.1 | 0.0 | 50.1 | 67.8 | 71.1 | 69.4 | 71.1 | 71.1 | 72.7 | 69.4 | 75.9 | 69.4 |
| Comp.Ex.2 | 0.0 | 40.1 | 57.0 | 60.1 | 58.6 | 58.6 | 55.5 | 55.5 | 50.9 | 46.8 | 41.2 |
| Comp.Ex.3 | 0.0 | 85.6 | 85.6 | 80.8 | 74.3 | 66.2 | 59.8 | 53.3 | 50.1 | 50.1 | 46.8 |
| Comp.Ex.4 | 0.0 | 67.8 | 77.5 | 79.1 | 74.3 | 74.3 | 71.1 | 71.1 | 66.2 | 66.2 | 59.8 |
| Comp.Ex.5 | 0.0 | 89.5 | 93.6 | 93.6 | 94.9 | 92.2 | 92.2 | 89.5 | 86.8 | 82.7 | 78.7 |
| Comp.Ex.6 | 0.0 | 43.5 | 51.5 | 58.4 | 58.9 | 55.4 | 52.3 | 55.6 | 53.0 | 58.8 | 53.6 |
| Comp.Ex.7 | 0.0 | 45.2 | 56.5 | 61.4 | 59.8 | 63.0 | 59.8 | 66.2 | 63.0 | 71.1 | 64.6 |
| Comp.Ex.8 | 0.0 | 32.3 | 46.8 | 51.7 | 51.7 | 54.9 | 51.7 | 58.1 | 54.9 | 64.6 | 56.5 |
| Comp.Ex.9 | 0.0 | 80.2 | 94.0 | 92.5 | 80.2 | 66.3 | 55.5 | 43.2 | 38.5 | 35.5 | 30.8 |

Figure 1 is a graph showing the relationship between the dissolution time and the dissolution percentage (%) of nifedipine. The graph shows plots of the results of Example 1 in which a composite was produced by subjecting a sold dispersion containing methyl cellulose and a drug at a mass ratio of 100:20 to addition of HPMCAS-1 at a mass ratio of 25, relative to mass ratio of 100 of the methyl cellulose; plots of the results of Comparative Example 1 in which a composite was produced in the same manner as in Example 1 except that the HPMCAS-1 was replaced by microcrystalline cellulose at a mass ratio of 25, relative to mass ratio of 100 of the methyl cellulose; plots of the results of Example 4 in which a composite was produced by subjecting a sold dispersion containing a VP/VA copolymer and a drug at a mass ratio of 100:20 to addition of HPMCAS-1 at a mass ratio of 25, relative to mass ratio of 100 of the VP/VA copolymer; and plots of the results of Comparative Example 4 in which a composite was produced in the same manner as in Example 4 except that the HPMCAS-1 was replaced by microcrystalline cellulose at a mass ratio of 25, relative to mass ratio of 100 of the VP/VA copolymer. These findings show that the presence of HPMCAS outside the solid dispersion greatly improves the dissolution of drug, including the initial dissolution. The improved dissolution of drug in the solid dispersion is influenced not only by the presence of the outer HPMCAS but also by the type of carrier.

When methyl cellulose (in Examples 1, 5, 6 and 7), HPMC (in Example 2), HPMCP (in Example 3), a vinylpyrrolidone-vinyl acetate copolymer (in Example 4) or HPMCAS-2 (in Example 8) was used as a carrier of solid dispersion, high dissolution was obtained in combination with HPMCAS-1 outside the solid dispersion. Surprisingly, the initial dissolution for the first 15 minutes was also significantly higher. High dissolution was also obtained in Example 5 in which griseofulvin was used in place of nifedipine as the drug. Good dissolution was also obtained in Example 8 in which the solid dispersion was produced not by a spray drying but by a hot-melt extrusion.

Comparative Example 8 in which methyl cellulose as the carrier of the solid dispersion was combined with HPMC outside the solid dispersion showed the dissolution inferior to that of Example 6 in which methyl cellulose as the carrier of the solid dispersion was combined with HPMCAS outside the solid dispersion.

## Claims

1. A composite comprising at least:
a solid dispersion comprising at least a drug and a carrier selected from the group consisting of a vinylpyrrolidone-vinyl acetate copolymer, methyl cellulose, hydroxypropyl methyl cellulose, hydroxypropyl methyl cellulose phthalate and first hydroxypropyl methyl cellulose acetate succinate; and
second hydroxypropyl methyl cellulose acetate succinate outside the solid dispersion.

2. The composite according to claim 1, wherein the carrier is methyl cellulose having a viscosity at 20°C of from 1.0 to 50.0 mPa·s, as determined in a 2% by mass aqueous solution with an Ubbelohde-type viscometer.

3. The composite according to claim 1 or 2, wherein an amount of the drug is 10 to 100 parts by mass and an amount of the second hydroxypropyl methyl cellulose acetate succinate is 10 to 100 parts by mass, relative to 100 parts by mass of the carrier.

4. The composite of claim 1 or claim 2, wherein the drug is a poorly water-soluble.

5. A method for producing a composite comprising at least steps of:
preparing a solid dispersion comprising at least a drug and a carrier selected from the group consisting of a vinylpyrrolidone-vinyl acetate copolymer, methyl cellulose, hydroxypropyl methyl cellulose, hydroxypropyl methyl cellulose phthalate and first hydroxypropyl methyl cellulose acetate succinate; and
adding second hydroxypropyl methyl cellulose acetate succinate to the solid dispersion.

6. The method for producing a composite according to claim 5, wherein the step of adding the second hydroxypropyl methyl cellulose acetate succinate to the solid dispersion comprises mixing the solid dispersion with the second hydroxypropyl methyl cellulose acetate succinate, or granulating the solid dispersion in the presence of the second hydroxypropyl methyl cellulose acetate succinate, or coating the solid dispersion with the second hydroxypropyl methyl cellulose acetate succinate.

7. The method for producing a composite according to claim 5 or claim 6, wherein the carrier is methyl cellulose having a viscosity at 20°C of from 1.0 to 50.0 mPa·s, as determined in a 2% by mass aqueous solution with an Ubbelohde viscometer.
